# EUROPEAN PATENT SPECIFICATION

(11) **EP 1 613 773 B1**
(45) Date of publication and mention of the grant of the patent: **10.02.2010**
(21) Application number: 04727930.2
(22) Date of filing: 16.04.2004
(51) Int. Cl.: C12Q 1/68

(54) **METHOD FOR CHARACTERISING POLYNUCLEOTIDES**
METHODE ZUR CHARAKTERISIERUNG VON NUKLEINSÄUREN
PROCEDE DE CARACTERISATION DE POLYNUCLEOTIDES

(30) Priority: 16.04.2003 GB 0308851
(43) Date of publication of application: 11.01.2006
(73) Proprietor: Lingvitae AS, 0372 Oslo (NO)
(72) Inventor: LEXOW, Preben, N-0372 Oslo (NO); RAGNHILDSTVEIT, Erlend, N-0372 Oslo (NO)
(74) Representative: Jappy, John William Graham
(86) International application number: PCT/GB2004/001673
(87) International publication number: WO 2004/094664

(56) References cited:
- EP-A- 0 701 001
- WO-A-00/39333
- WO-A-00/60124
- WO-A-01/61036
- US-A- 5 714 330
- US-B1- 6 258 533
- US-B1- 6 258 539

## Description

### Field of the Invention

This invention relates to methods for converting a target polynucleotide into readable information chains, for subsequent determination of the sequence of the target polynucleotide.

### Background to the Invention

Advances in the study of molecules have been led, in part, by improvement in technologies used to characterise the molecules or their biological reactions. In particular, the study of the nucleic acids DNA and RNA has benefited from developing technologies used for sequence analysis and the study of hybridisation events.

The principal method in general use for large-scale DNA sequencing is the chain termination method. This method was first developed by Sanger and Coulson (Sanger et al., Proc. Natl. Acad. Sci. USA, 1977; 74: 5463-5467), and relies on the use of dideoxy derivatives of the four nucleotides which are incorporated into the nascent polynucleotide chain in a polymerase reaction. Upon incorporation, the dideoxy derivatives terminate the polymerase reaction and the products are then separated by gel electrophoresis and analysed to reveal the position at which the particular dideoxy derivative was incorporated into the chain.

Although this method is widely used and produces reliable results, it is recognised that it is slow, labour-intensive and expensive.

US-A-5302509 discloses a method to sequence a polynucleotide immobilised on a solid support. The method relies on the incorporation of 3-blocked bases A, G, C and T having a different fluorescent label to the immobilised polynucleotide, in the presence of DNA polymerase. The polymerase incorporates a base complementary to the target polynucleotide, but is prevented from further addition by the 3'-blocking group. The label of the incorporated base can then be determined and the blocking group removed by chemical cleavage to allow further polymerisation to occur. However, the need to remove the blocking groups in this manner is time-consuming and must be performed with high efficiency.

WO-A-00/39333 describes a method for sequencing polynucleotide by converting the sequence of a target polynucleotide into a second polynucleotide having a defined sequence and positional information contained therein. The sequence information of the target is said to be "magnified" in the second polynucleotide, allowing greater ease of distinguishing between the individual bases on the target molecule. This is achieved using "magnifying tags" which are predetermined nucleic acid sequences. Each of the bases adenine, cytosine, guanine and thymine on the target molecule is represented by an individual magnifying tag, converting the original target sequence into a magnified sequence. Conventional techniques may then be used to determine the order of the magnifying tags, and thereby determining the specific sequence on the target polynucleotide.

In a preferred sequencing method, each magnifying tag comprises a label, e.g. a fluorescent label, which may then be identified and used to characterise the magnifying tag.

Although the method disclosed in this patent publication has many advantages, there is still a need for improved methods for sequencing target polynucleotides.

### Summary of the Invention

According to a first aspect of the present invention, a method for identifying specific characteristics of a target polynucleotide present in a sample, comprises the steps of:
i) attaching to one end of each target polynucleotide in the sample a polynucleotide signal sequence that is specific for the characteristic under study;
ii) contacting the target polynucleotides with a molecule that interacts with the target polynucleotide if the characteristic is present;
iii) separating those target polynucleotides that interact from those that do not;
iv) optionally repeating steps (i) to (iii); and
(v) identifying which signal sequences are present on the separated target polynucleotides, and in which order, to thereby determine the characteristics of each target polynucleotide.

The method of the present invention permits multiple characteristics of a target polynucleotide to be determined in a simple manner that can be readily automated. In the preferred embodiment, the method relies on the incorporation of two polynucleotides molecules onto those targets having the specific characteristic, allowing an amplification reaction to be performed, to thereby selectively increase the number of target polynucleotides with the desired characteristic and to effectively remove those targets lacking the characteristic. The method is particularly suitable for determining the sequence of a target polynucleotide.

According to a second aspect of the invention, a method for determining the sequence of a target polynucleotide, comprises the steps of:
i) treating a sample of a double-stranded target polynucleotide to create overhangs at the 5' and 3' ends, each with a defined number of bases;
ii) dividing the sample and contacting each separate sample with a double-stranded polynucleotide signal sequence and a double-stranded adapter polynucleotide, each signal sequence representing a specific polynucleotide sequence at the 5'-overhang, and comprising an overhang that permits hybridisation and ligation to the 3' end of the target polynucleotide, and each adapter comprising an overhang that is of complementary sequence to that of the sequence represented by the signal sequence;
iii) carrying out the polymerase reaction on the sample(s) using primers that hybridise to the signal sequence and adapter sequence, wherein the product of the polymerase reaction comprises a restriction site that permits cleavage of the adapter and that part of the target polynucleotide that formed the 5'-overhang, to form a new 5'-overhang, optionally repeating steps (I) to (iii) using restriction enzymes to create the overhangs; and
iv) identifying which signal sequences are present on the amplified products, and in which order, to thereby determine the sequence of the target polynucleotide.

### Description of the Drawings

The invention is described with reference to the accompanying figures, wherein:
Figure 1 is a schematic representation of a signal sequence, illustrating the conversion of part of a target polynucleotide into a defined signal sequence;
Figure 2 is a schematic representation of a polynucleotide sequencing experiment carried out using the present invention;
Figure 3 is a schematic representation of the use of different compartments with associated specific signal sequences in a conversion process;
Figure 4 shows the results of conversion, with three cycles being performed to form a series of signal sequences representing the base sequence on a target polynucleotide;
Figure 5 is a schematic representation of a polynucleotide sequencing experiment, carried out to determine a three base sequence per cycle; and
Figure 6 shows the results of conversion, demonstrating the incorporation of specific signal sequences onto a target polynucleotide.

### Description of the Invention

The method of the present invention is an adaptation of the conversion method disclosed in WO-A-00/39333. In summary, the present invention permits specific characteristics of a target polynucleotide to be identified based on the incorporation of specific signal sequences. The signal sequences are incorporated onto all the target polynucleotides present in a sample, but only those that comprise the specific characteristic are separated and characterised.

The method is particularly suitable for determining the sequence of a target polynucleotide. Using the method, a defined number of sequences at one end of the target polynucleotide can be converted into a defined signal sequence at the other end of the target polynucleotide. Through a series of steps, the "sequenced" end of the target is cleaved, while the signal sequence end is built up, generating a defined series of signal sequences which can be determined through a subsequent identification step.

The term "polynucleotide" is well known in the art and is used to refer to a series of linked nucleic acid molecules, e.g. DNA or RNA. Nucleic acid mimics, e.g. PNA, LNA (locked nucleic acid) and 2'-O-methRNA are also within the scope of the invention.

The reference herein to the bases A, T(U), G and C, relate to the nucleotide bases adenine, thymine (uracil), guanine and cytosine, as will be appreciated in the art. Uracil replaces thymine when the polynucleotide is RNA, or it can be introduced into DNA using dUTP, again as well understood in the art.

Reference is made herein to the 5' and 3' ends of the target polynucleotide. In certain embodiments the target polynucleotide is double-stranded, and so each end comprises both 5' and 3' ends. However, the reference to 5' and 3' ends is made with reference to the strand of the target that is under study or is being sequenced. It will also be apparent that where the specification refers to the 5' end as the end being sequenced, it will be possible to modify the procedure so that it is the 3' end that is sequenced, i.e. the signal sequences and adapters ligate to opposite ends to those indicated herein.

In its broadest aspect, the invention allows the identification of specific characteristics of the target polynucleotide. A characteristic may be the sequence of the polynucleotide. Alternatively, a characteristic may be the ability of the target polynucleotide to bind to other molecules, for example to DNA-binding proteins or to hybridise to complementary polynucleotides. The method may also be used to identify and characterise restriction enzyme sites present within the target polynucleotide sequence.

Each characteristic is identified by carrying out a reaction with a molecule that interacts with a target polynucleotide having the characteristic and discriminates from those lacking the characteristics. Those target polynucleotides that interact with the molecule are labelled with a signal sequence that is specific for the characteristic and permits the characteristic to be determined in a later read-out step.

The present invention relies on the incorporation of a signal sequence on to all target polynucelotides in a sample, with the signal sequence representing a specific characteristic under study. The target polynucleotides are then contacted with a molecule and those that interact are separated. In this way, it is possible to identify the characteristic on identification of the signal sequence.

The separation of those target molecules that interact with the molecule can be carried out in various ways. For example, the molecule may contain a binding moiety that permits separation of the target polynucleotide/molecule complex. Sutiable binding moieties include antibodies and biotin/streptaridin. Alternatively, size separation may be carried out, as it will be possible to distinguish the target/molecule complex from non-complexed components.

The preferred embodiment of the present invention relies on the incorporation of a polynucleotide signal sequence and polynucleotide adapter sequence onto the target polynucleotide, which permits an amplification reaction to be carried out to amplify those target polynucleotides which have reacted with a specific molecule. The resulting products of the polymerase reaction may be further characterised by repeating the steps of reacting the target with a molecule and incorporating a further signal sequence representing the characteristic under study.

The method of the invention is preferably carried out using multiple reaction compartments, one for each putative characteristic being studied. Each reaction compartment has an associated signal sequence specific for that compartment. The sample containing the target polynucleotides is divided between the reaction compartments and a signal sequence is attached, for example by ligation, to one end of each target polynucleotide. The signal sequence is attached to all of the target polynucleotides in the compartment, irrespective of whether the target has the specified characteristic. The next step is to test for the characteristic by reacting each target with a molecule that interacts specifically with those targets that comprise the characteristic. The molecule may therefore be a polynucleotide-binding protein, enzyme or other polynucleotide. Those targets that interact with the molecules are then separated. In the preferred embodiment, of the molecule comprises an adapter sequence. Therefore, on interaction between the target and the molecule, the molecule (comprising the adapter) is attached or ligated to the target. Those targets that comprise both a signal sequence and adapter are then amplified.

The amplification reaction may be carried out using any suitable method. The preferred method is to use a conventional polymerase reaction using conditions known in the art. It will be appreciated that other amplification reactions can also be carried out, including isothermal pcr and amplification in bacteria.

A preferred embodiment is to use the method to sequence the target polynucleotide. This is carried out by identifying a defined number of bases, e.g. three or four bases, at one end of the target polynucleotide, and then converting this information into a signal sequence on the opposite end, thereby permitting additional conversion cycles to take place, without the removal of incorporated signal sequences from previous cycles.

The sequencing method may be carried out by obtaining the target polynucleotide and treating this with a series of restriction enzymes to form defined overhang regions at each end of the target polynucleotide, which allow incorporation of a signal sequence and an adapter during each conversion step.

A "signal sequence" is a single-stranded or double-stranded polynucleotide that comprises distinct "units" of nucleic acid sequence. Each characteristic, eg., each of the bases A, T(U), G and C on the target is represented by a distinct and predefined unit, or unique combination of units. Each unit will preferably comprise two or more nucleotide bases, preferably from 2 to 50 bases, more preferably 2 to 20 bases and most preferably 4 to 10 bases, e.g. 6 bases. There are at least two different bases contained in each unit. In a preferred embodiment, there are three different bases in each unit. The design of the units is such that it will be possible to distinguish the different units during a "read-out" step, e.g. involving either the incorporation of detectably labelled nucleotides in a polymerisation reaction, or on hybridisation of complementary oligonucleotides. For example, if the characteristic being studied is the sequence of the target each base on the target is represented by a specific series of bases in a unit.

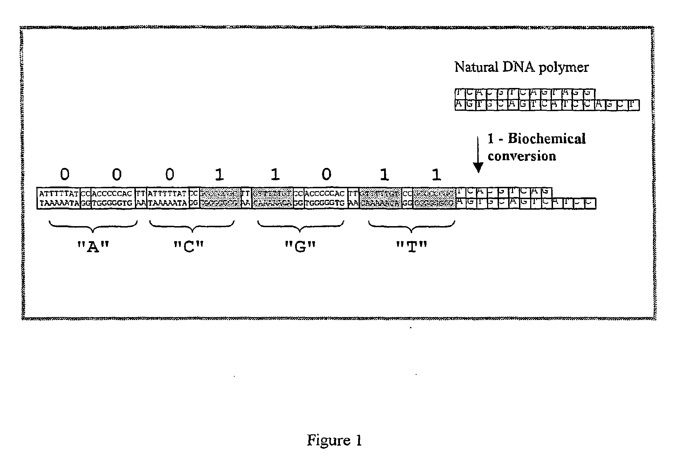

In a preferred embodiment, each signal sequence comprises two units of distinct sequence which represent all of the four bases on the target. According to this embodiment, the two units can be used as a binary system, with one unit representing "0" and the other representing "1 ". Each base on the target is characterised by a combination of the two units. For example, adenine may be represented by "0" + "0", cytosine by "0" + "1", guanine by "1" + "0" and thymine by "1" + "1", as shown in Figure 1. It is necessary to distinguish between the units, and so a "stop" signal can be incorporated into each unit. It is also preferable to use different units representing "1" and "0", depending on whether the base on the target (template) polynucleotide is in an odd or even numbered position.

This is demonstrated as follows:
Odd numbered template sequence:
   "0" : ATTTTTAT(CC)
   "1" : GTTTTTGT(CC)
Even numbered template sequence:
   "0" : ACCCCCAC(TT)
   "1" : GCCCCCGC(TT)

Suitable signal sequences are also described in WO-A-00/39333.

The "adapter" is a single-stranded or double-stranded polynucleotide. The purpose of the adapter is to provide the necessary sequence to permit amplification to occur. In a preferred embodiment, the adapter is a double-stranded polynucleotide that comprises a defined overhang that is capable of hybridising and ligating to a complementary region on the target polynucleotide. The complementary region is usually at the end of the target opposite that to which the signal sequence incorporates. The adapter provides the necessary primer binding site necessary in the amplfication reaction. In a preferred embodiment, the adapter is designed to hybridise to that part of the target polynucleotide that is being sequenced, i.e. the 5'-overhang part of the target polynucleotide. If the 5'-overhang on the target is 4 bases, then the complementary overhang on the adapter will also be 4 bases. This permits ligation to occur to form a double-stranded polynucleotide. Given that the 5'-overhang of the target is of unknown sequence, it will usually be necessary to use a combination of adaptors having all permutations of the (for example) 4 base sequence. The adapters of different sequence will be used in separate reactions, as discussed below.

Each adapter will usually have a restriction enzyme recognition site incorporated, allowing its cleavage during a later step. This can be used to create a new 5'-overhang.

The sequencing method of the preferred embodiment comprises the following general steps:

### Digestion

A sample of the target polynucleotide is first treated with one or more restriction enzymes, resulting in an overhang in the target with a defined number of bases, e.g. 3 bases, that is used for ligation of signal sequences later in the procedure (the overhang). An overhang is also created at the opposite end of the target polynucleotide (the 5'-overhang). It is this overhang that is to be identified (represented) by the signal sequence during the conversion process and is to be ligated to the adapter.

### Ligation

The sample with the target polynucleotide is divided evenly into reaction compartments representing all permutations of the overhang to be sequenced. For example, if the overhang to be sequenced is 4 bases, then 256 reaction compartments are to be used. In the first compartment, the target polynucleotide is contacted with a signal sequence that represents for example AAAA (when the overhang to be sequenced is 4 bases), the signal sequence being ligated to the 3'-overhang on all the target polynucleotides. An adapter polynucleotide with the overhang TTTT is also added, but this will only ligate specifically to the target polynucleotides which contain an AAAA overhang, leaving polynucleotides with a different overhang sequence with an unligated overhang. The same procedure is performed in the other compartments, each representing a different combination of the possible 4 base sequence. In each case the adapter will be the complement of the sequence represented by the signal sequence.

Although it is preferable for the signal sequence to represent all of the bases of the overhang, it is possible to sequence fewer bases than those present in the overhang. For example, if the overhang comprises 4 bases, the signal sequence may be used to represent 3 of these. This is illustrated in the Examples disclosed below.

### Amplification

After the ligation steps have been carried out, the samples can be pooled, and a polymerase reaction carried out. The polymerase reaction is carried out using primers which target the ends of the signal sequence and adapter, and therefore only those molecules that have successfully ligated both the signal sequence and the adapter sequence will be amplified exponentially, while those molecules containing only a signal sequence will be removed as a consequence of linear amplification. The result will be a population of converted polynucleotide fragments, where the 4 base overhang that was initially generated at one end of the target polynucleotide has been replaced with a signal sequence representing the 4 bases at the other end of the target polynucleotide.

In a preferred embodiment, the polymerase reaction is carried out using methyl-dCTP, which ensures that native restriction enzyme sites remain inactive. Additional restriction enzyme sites are incorporated by the adapter molecules, thereby permitting further conversion cycles to occur. The primers used in the polymerase reaction are specific for sequences found within the signal sequence and adapter, thereby ensuring that the amplification step occurs only when both the signal sequence and adapter is ligated onto the target polynucleotide.

It is also preferable for the primer binding sites to be different in the different sets of signal sequence and adapter, ie., the signal sequence and adapter used in one conversion cycle will comprise different primer binding sites from those used in an earlier cycle. This ensures that the correct sequence is amplified.

The overhangs may be produced by different means, although the preferred embodiment is to use restriction enzymes, e.g. class IIs restriction enzymes. These enzymes exhibit no specificity to the sequence that is cut and they can therefore generate overhangs with all types of base compositions. The binding site of the restriction enzyme can be located so that an overhang is formed inside the actual target polynucleotide. In practice, it is preferable to choose enzymes that generate 3-4 base pair overhangs.

Class IIs restriction endonucleases are known, and are identified in WO-A-00/39333.

In one embodiment, the target polynucleotide is first treated with an adapter polynucleotide which incorporates a recognition sequence for the restriction enzyme Bbvl, and the polynucleotide is then treated with the restriction enzyme to generate a specific overhang at the 5' end. Native recognition sites for BbvI are inactivated by first treating the target polynucleotide by methylation. Having created the first overhang, subsequent overhangs at the 5' end are preferably created using the restriction enzyme *Sfa*NI. This is carried out by ligating a specific adapter sequence at the 5' end which incorporates the recognition sequence for *Sfa*NI.

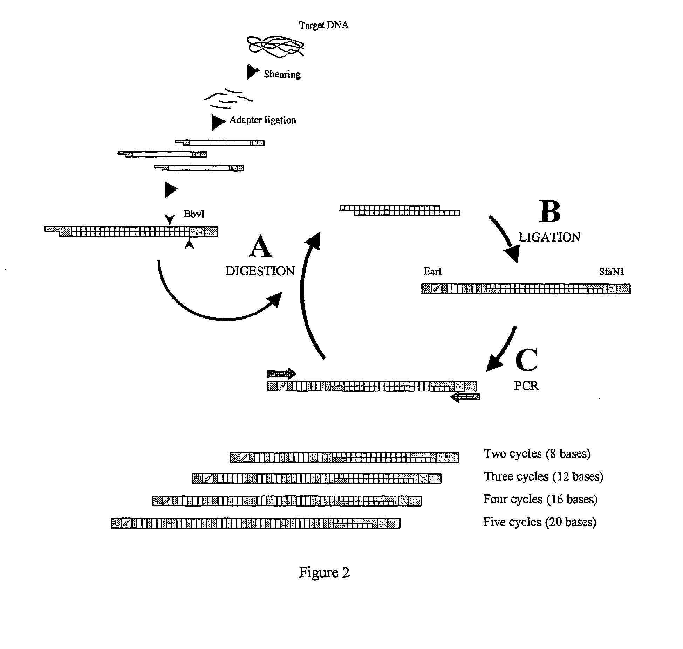

The initial overhang at the 3' end is preferably created by ligating a polynucleotide that incorporates a defined overhang. The defined overhang permits a first signal sequence to hybridise and ligate at the 3' end. In another embodiment, the target polynucleotide is first treated with an adapter polynucleotide which incorporates a recognition sequence for the restriction enzymes Mmel and Earl (or its isoschizomer Eam 11041). The polynucleotide is then treated with the restriction enzyme Mmel to cleave off 20 basepairs from the target molecule and associate it with the adapter polynucleotide. A second adapter polynucleotide, containing a recognition sequence for the restriction enzyme SfaNl, is then ligated to the adapter-associated target polynucleotide via the Mmel generated overhang. A PCR amplification is carried out to amplify the fragments containing the 20 bp target polynucleotide flanked by two adapter polynucleotides. To protect internal sites from subsequent Earl and SfaNl digestion, the PCR is carried out with methylated dCTP replacing normal dCTP in the dNTP mix. Amplification of target polynucleotides flanked by Mmel generated overhangs (i.e., by-products from the digestion), can be eliminated by using unphosphorylated second adapters. The specific overhang at the 5' end is created by digesting the PCR product with the restriction enzyme SfaNI (or equivalents). The defined overhang at the 3' end is created by digesting the PCR product with the restriction enzyme EarI (or Eam11041) The signal sequence incorporates a restriction enzyme site, which is preferably for the restriction enzyme *Ear*I. Subsequent cycles of cleavage and incorporation occur using the restriction enzymes *Ear*I and *Sfa*NI, as shown in Figure 2.

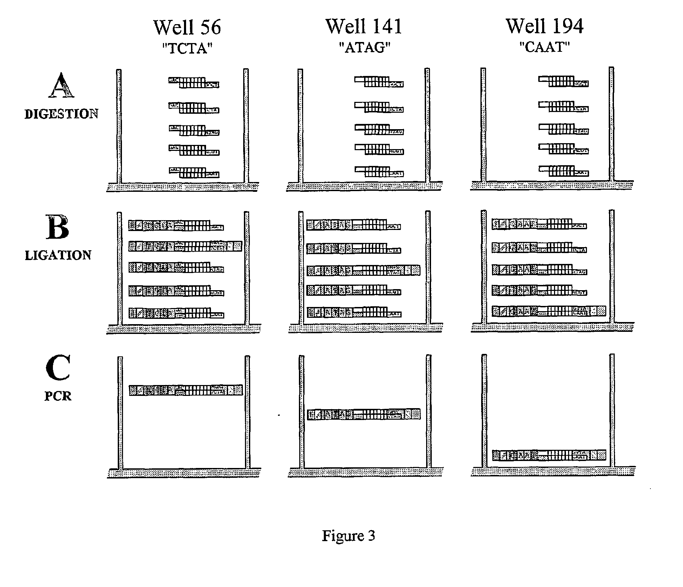

The conversion cycle is further illustrated in Figure 3, which shows the fate of five different fragments within three arbitrarily picked reaction compartments out of the 256 compartments used in the procedure. The fragments are first evenly distributed into the 256 compartments. In compartment 56, where the overhang "TCTA" is to be identified, a signal sequence representing this base composition is introduced and ligated with the 3 base 3'-overhang on the left end of the target polynucleotide. This ligation occurs regardless of the composition of the 4 base 5'-overhang on the right side of the polynucleotide. A specific adapter, that only ligates with fragments with 3' TCTA -5' overhangs, is then introduced and ligated. Mismatch ligation of the adapter can be reduced by performing the ligation in presence of "blocking" adapters. Blocking adapters have a different overhang sequence than the specific adapter and hence ligate to target polynucleotides having a non-complementary overhang to the specific adapter. A final amplification reaction then selectively amplifies the fragments that have ligated with the specific adapter and hence removes the other fragments which have been associated with an incorrect signal sequence. The same procedure is performed in compartments 141 and 194 with the exception that the composition of the signal sequences and the adapters are adjusted in accordance with the overhangs that are to be identified in the wells (3'-ATAG-5' and 3'-CAAT-5').

The target polynucleotides from the 256 compartments are preferably pooled into one common reaction tube before the amplification reaction takes place, thereby avoiding the inconvenience of performing 256 separate amplification reactions.

After subsequent conversion cycles are carried out, the sequence at the 5' end of the target polynucleotide is reduced and the signal sequences introduced at the 3' end of the target polynucleotide are increased in a defined way corresponding to the now "sequenced" target polynucleotide. Determining the type and order of the signal sequences may be carried out using methods disclosed in WO-A-00/39333, or in the co-pending International Patent Application filed in the name of LingVitae and claiming priority from GB0308852.3.

The amplification reaction may produce unwanted artefacts and various steps can be taken to reduce or remove these. For example, it is preferable if the primer recognition site on different signal sequences (and optionally also different adapters) is different, to reduce the likelihood of primers binding to internal signal sequences and amplifying fragments of the larger converted target.

It is also preferable to design the signal sequences so that consecutive or subsequent signal sequences comprise distinct base sequences. This ensures that primers introduced to hybridise to the terminal signal sequence, do not hybridise to internal signal sequences, resulting in fragments being produced during the amplification step.

Incomplete digestion by one or more of the restriction enzymes may also be a source of artefacts. Utilising restriction enzymes having high specificity and activity will reduce this problem.

Artefacts may also be removed by gel purification, or by immobilising the products prior to digestion with the restriction enzyme; those products not completely digested will remain immobilised and can be separated from those products that have undergone complete digestion.

The removal of excess adapters and targets prior to amplification will also reduce the production of artefacts.

The following Examples illustrate the invention.

### Example 1

### Initial preparation using BbvI and no size restriction

Prior to (or after) DNA fragmentation the target polynucleotide is methylated to inactivate native recognition sites. Using BbvI as the restriction enzyme, M.Bbv (Megabase Research Products) is used to inactivate its native sites. The target polynucleotide is randomly fragmented into pieces of 500-1000 base pairs by mechanical shearing using a HydroShear device (GeneMachines). In combination with sonication, even shorter fragments can be generated. To increase the efficiency of the subsequent step of blunt-end adapter ligation, the sheared polynucleotides are optionally treated with DNA polymerase to fill in or remove overhangs using its exonuclease function. T4 DNA polymerase and Klenow Large fragment alone or in combination are commonly used for this purpose.

Two sorts of polynucleotide adapters are blunt-end ligated to the target polynucleotide fragments. One incorporates a BbvI site and the other incorporates an overhang for subsequent ligation to a signal sequence. Each adapter has a length of 10-20 base pairs and is added in a 10X molar excess to the target polynucleotides. The adapters are phosphorylated except for one of the strands of the Bbvl adapter. In addition, the opposite strand of this adapter carries a 3' deoxy group to prevent concatemerization during ligation. The ligation is performed under optimized conditions for blunt-end ligation following manufacturers recommendations.

The ligated polynucleotide fragments are digested with BbvI. After digestion, a size selection (e.g., gel separation or column fractionation) is performed to remove excess adapters, cleaved end fragments and polynucleotide fragments below and above a certain threshold value (e.g., two-fold size distribution).

### Example 2.

### Initial preparation using Mmel and size restriction

The target polynucleotide is randomly broken into pieces of 500-1000 base pairs by mechanical shearing using a HydroShear device (GeneMachines). Together with sonication, shorter fragments can be generated. To increase the efficiency of the subsequent step of blunt-end adapter ligation, the sheared polynucleotides are optionally treated with DNA polymerase to fill in or remove overhangs as discussed in Example 1. A 10X molar excess of a 20-40 bp blunt-end adapter is ligated to the fragmented target polynucleotides. The ligation is performed under optimized conditions for blunt-end ligation following the manufacturer's recommendations. Only the anti-sense strand of the adapter is phosphorylated to ensure unidirectional ligation (in addition the anti-sense strand incorporates a 3' dideoxy group or blocking group to prevent concatemerization). The adapter carries at its very 3' end a restriction site for Mmel and a restriction site for Earl (or Eam1104I) further upstream. Size exclusion or affinity spin columns (e.g., MinElute PCR clean-up from Qiagen) are conveniently used to remove excess adapters as well as changing buffer.

Mmel digestion is performed under optimal conditions to cleave 20 basepairs from the target molecule and associate it with the adapter polynucleotide. A spin column is used to change buffer (e.g. BioSpin-6 from BioRad). A second adapter polynucleotide, containing a recognition sequence for the restriction enzyme SfaNI, is then ligated to the adapter-associated target polynucleotide via the Mmel generated overhang (because Mmel generates a two bases 3' overhang, the second adapter is a pool of adapters having all the 16 permutations of two bases in the 3' overhang). The SfaNl site is positioned to cut into the terminal four bases of the target sequence. The adapter is kept unphosphorylated to eliminate subsequent amplification of target polynucleotides containing two Mmel generated overhangs (a byproduct from the target polynucleotide digestion). A PCR amplification is carried out to amplify the fragments containing the 20 bp target polynucleotide flanked by two adapter polynucleotides. To protect internal sites from subsequent Earl and SfaNl digestion, the PCR is carried out with methylated dCTP replacing normal dCTP in the dNTP mix.

A PCR clean-up spin column is used to remove excess primers, DNA polymerase and to change buffer.

The PCR amplified material is digested with Earl (or Eam11041) and SfaNl to generate the overhangs needed for signal sequence ligation and for biochemical conversion. By using biotinylated primers in the PCR, digested end fragments can be removed by means of affinity (e.g., streptavidin coated beads).

### Example 3.

### The principle of cyclic conversion where four nucleotides are converted per cycle

The principle of cyclic conversion of nucleic acid bases into readable signal sequences was demonstrated by converting a sequence of 12 bases in a target DNA into its corresponding signal chain. The conversion took place in steps through a cyclic method, where 4 bases were converted in each cycle for a total number of 3 cycles. The end product was a signal chain consisting of 12 signal components representing the relevant base sequence in the target DNA. The experiment is described schematically in Figure 2.

A target DNA fragment of 240 bp, containing internal sites for *Sfa*NI and *Eam*1104I was PCR-amplified from the bacteriophage Lambda genome (1507-1703) using primers containing restriction sites for *Eam*1104I and *Sfa*NI (the top fragment in Figure 2) respectively. In order to avoid cutting from the internal sites later, the PCR reaction was conducted with methylated deoxy-Cytosine nucleotides (m5-dCTP) instead of the usual deoxy-Cytosine (dCTP). PCR conditions: (50 µl): 10 mM KCI, 10mM (NH₄)₂SO₄, 20mM Tris-HCl, 0.1 % Triton-X-100, pH 8.8 (Thermopol buffer, New England Biolabs), 2 mM Mg²⁺, 200 µM dNTP (- dCTP) and 200 µM m5-dCTP (Amersham Pharmacia Biotech), 20 pmol Lambda primer #328 (5'-agactggcgatccctggcatcacccctccagcgtgttttat-3'; SEQ ID No 1) and 20 mpol Lambda primer #329 (5'-gcactgataggcgtcactcttcgctgtacgctglccagatgt-3'; SEQ ID No 2) (MWG biotech), 10 ng Lambda genome, 1 U Vent polymerase (New England Biolabs). The PCR cycling was conducted with a PTC-200 (MJResearch). Hot start: 95°C, 5 minutes, 35 cycles consisting of: 95°C, 15 seconds, 58°C, 20 seconds, 72°C, 30 seconds. Complete extension step: 72°C, 5 minutes.

### Cycle 1:

The fragment enters the sequencing cycle (Figure 2, A) and is cut with *Eam*1104I and *Sfa*NI (as the internal sites are methylated and protected against cutting, cutting will only take place from the primer areas). Digestion conditions: 100 mM NaCl, 50 mM Tris-HCl, 10 mM MgCl₂, 1 mM DTT, pH 7.9 (NEB3, New England Biolabs), 1 µg methylated PCR fragment, 20 U *Eam*1104I, 3U *Sfa*NI, and incubation for one hour at 37°C. The enzymes were heat-inactivated at 65°C for 20 minutes. The cut fragment was cleaned using GibcoBRL PCR purification system (Gibco) and then eluted with 10 mM Tris-HCI. The cut fragment corresponds to the nature of those fragments that are obtained after an initial preparation or one cycle of conversion (described in Example 1) in that it has an overhang of 3 bases for signal chain ligation and an overhang of 4 bases for ligation to a specific right hand adapter.

The conversion step where the selection marker is attached (Figure 2, B) was carried out by ligating the fragment with the specific adapter and its associated signal sequence: 40 mM Tris-HCl, 10 mM MgCl₂, 10 mM DTT, 0.5 mM ATP, pH 7.8 (Ligase buffer, Fermentas), 1.6 pmol adapter1 (5'-Biotin-ggctaggtgctgatgaacgcatcg-3; SEQ ID No 3' annealed to 5'-tggacgatgcgttcatcagcacctagcc-3'; SEQ ID No 4 (MWG-biotech), 1.9 pmol signal sequence 1 (1), 7.5 Weiss-U T4 DNA ligase (Fermentas). The incubation took place at room temperature (22°C) for 2.5 hours. The ligase was heat-inactivated at 65°C for 10 minutes.

The signal sequences used in the experiment were made in the following way: Signal components (each representing one base) were made by annealing oligonucleotides (approx. 45 bases) which were ligated together to create the desired signal sequences (the signal components are constructed with different overhangs which dictate the sequence of ligation). The signal sequences were amplified by PCR and cut with *Sap*I in order to generate the complementary overhang necessary for ligation of the signal sequence to the target DNA. Since all target DNA fragments have the same overhang for signal sequence ligation, all fragments in a given reaction will be ligated to the same signal sequence, irrespective of the sequence of the *Sfa*NI-generated overhang. Specificity and selection are therefore located in the adapter. As this ligates to that terminus of the target DNA which was cut with *Sfa*NI, only fragments with complementary overhang to the adapter will be ligated. A conversion therefore presupposes that the target DNA, as well as being ligated to a signal sequence, also has an overhang complementary to the adapter in order for ligation to this to take place. In other words, the adapter ensures a specific selection of fragments with an overhang sequence corresponding to the ligated signal sequence.

The selection step of the base conversion consists of selection and amplification of fragments which have had ligated to them both a signal sequence and a specific adapter (Figure 2,C). This was conducted using PCR under the following conditions (50 µl): 10 mM KCI, 10 mM (NH₄)₂SO₄, 20 mM Tris-HCI, 0.1 % Triton-X-100, pH 8.8 (Thermopol buffer, New England Biolabs), 2 mM Mg²⁺, 200 µM dNTP (-dCTP) and 200 µM m5-dCTP (Amersham Pharmacia Biotech), 20 pmol adapter1-primer #332 (5'-Biotin-ggctaggtgctgatgaacgcatcg-3'; SEQ ID No 5) and 20 pmol Signal sequence 1-primer #340 (5'-taatacgactcactatagcatgactcgagcctcttcgcga-3'; SEQ ID No 5) (MWG-biotech), approx. 3.5 fmol ligated target DNA and 1U Vent polymerase (New England Biolabs). PCR cycling was conducted with a PTC-200 (MJResearch). Hot start: 95°C, 2 minutes, 20 cycles consisting of: 95°C, 15 seconds, 66°C, 20 seconds, 72°C, 30 seconds. Complete extension step: 72°C, 5 minutes. Well 2 in Figure 2 shows the results of the initial cycle of the sequencing method. The correct fragment of 380 bp was generated.

### Cycle 2:

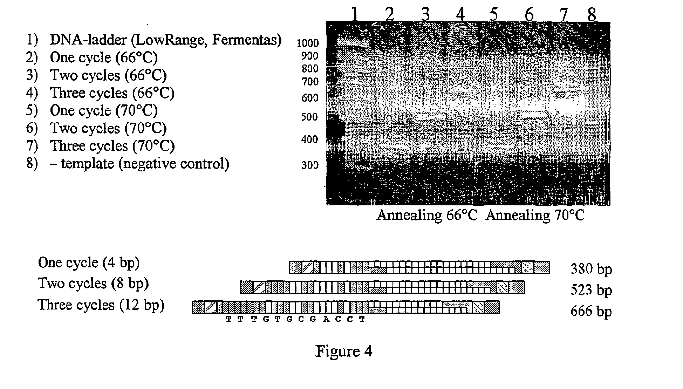

In order to convert the next 4 bases, the PCR product was cleaned with the GibcoBRL purification system (Gibco) and then cut with *Sfa*NI and *Eam*1104I. It is possible for this to take place as the signal sequence and the adapter from the initial cycle contain a site for *Eam*1104I and *Sfa*NI respectively. As the sites were located in the primer region, they are not blocked by methylation during PCR. To increase the efficiency of the cutting reaction, the cuts were conducted serially under optimal cutting conditions. *Sfa*NI cutting: 100 mM NaCl, 50 mM Tris-HCl, 10 mM MgCl₂, 1 mM DTT, pH 7.9 (NEB3, New England Biolabs), 1 µg methylated PCR fragment, 4U *Sfa*NI. Incubation at 37°C for 1 hour. Heat inactivation of the enzyme at 65°C for 10 minutes. A Micro Bio-Spin 6 column (BioRad) was used to clean the fragment. *Eam*1104I cutting: 33 mM Tris-acetate, 10 mM magnesium-acetate, 66 mM potassium-acetate, 0.1 mg/ml BSA, pH 7.9 (Tango Y⁺, Fermentas), SfaNl-digested PCR fragment, 10U *Eam*1104I. Incubated at 37°C for 1 hour, followed by heat inactivating at 65°C for 20 minutes. The digested product was cleaned by using the GibcoBRL purification system (Gibco). The initial step of the conversion was conducted corresponding to the initial cycle, except that a new signal sequence and its associated specific adapter (corresponding to the next 4 bases of the sequence) were added. To reduce potential carry-over problems, signal sequence 2 was designed with an different overhang from signal sequence 1. Ligation conditions: 40 mM Tris-HCI, 10 mM MgCl₂, 10 mM DTT, 0.5 mM ATP, pH 7.8 (Ligase buffer, Fermentas), 0.5 pmol adapter2 (5'Biotin-cgacagtacgacggaccagcatcc-3'; SEQ ID No 6 annealed to 5'-acgcggatgctggtccgtcgtactgtcg-3'; SEQ ID No 7) (MWG-biotech), 1 pmol signal sequence 2 (2), 1 Weiss-U T4 DNA ligase (Fermentas). The incubation took place at room temperature (22°C) for 1.5 hours. The ligase was heat-inactivated at 65°C for 10 minutes. PCR amplification took place under the same conditions as the initial cycle, except that a new primer set ( adapter2-primer #347, 5'-Biotin-cgacagtacgacggaccagcatcc-3; SEQ ID No 6' and signal sequence 2-primer #343, 5'-taatacgactcactatagcatcgaatgaccgcctcttccact-3'; SEQ ID No 8). Different primer sets for each sequencing cycle are preferable in order to minimise the danger of amplifying any remains from the previous sequencing cycle. The result of the PCR amplification in the second cycle is shown in Well 3 in Figure 4. The correct fragment of 523 bp was generated.

### Cycle 3.

Conversion of the next 4 bases and the PCR amplification followed the same pattern as described for Cycle 2: After a serial cutting with *Sfa*NI and *Eam*1104I, the fragment was ligated with signal sequence 3 (3) and adapter3 (5'-Biotin-atcgagcctggcatagcagcatca-3'; SEQ ID No 9 annealed to 5'-aaactgatgctatgccaggctcgat-3; SEQ ID No 10) (MWG-Biotech). The PCR amplification was conducted with the primer set: the adapter3-primer #353 (5'-atcgagcctggcatagcagcatca-3'; SEQ ID No 9) and signal sequence 3-primer #345 (5'-taatacgactcactatagcaccgggcaggatagactcttcaggt-3'; SEQ ID No 11). The result of the amplification in Cycle 3 is shown in Well 4 in Figure 4. Well 4 shows that two weak and one relatively strong bands are formed. The strong band comes closest to the expected length of 666 bp. The weak bands of the wrong size which can be seen in Well 4 (Cycle 3) and Well 3 (Cycle 2) are most probably the result of carry-over problems and mispriming. The weak band in Well 3 and the weak bands further down in Well 4 correspond to the size of the PCR fragment in the previous cycle. A possible explanation is that incomplete cutting of fragments in one cycle may function as a template in the next PCR cycle. Even though new primer sets in each cycle reduce this problem somewhat, the danger of mispriming is still present because of the enzyme sites the primers have in common. However, this type of mispriming may be eliminated by using more stringent annealing conditions during PCR (e.g. Mg²⁺, temperature), the use of more discriminating polymerases, moving the *Eam*1104I site away from 3' on the sequence chain primer, choice of new sequences or immobilisation of the fragments (e.g. the biotin-streptavidin system on microbeads) before cutting so that those fragments entering the next cycle are guaranteed to have been cut. The best solution for other types of mispriming would be to choose new primer sequences and/or optimise PCR conditions. Mispriming events that are caused by the presence of superflous DNA material (e.g., excess adapters or non-ligated DNA) can be removed by using a strategy of thio-protecting the recessed strand of the adapter. Digestion with a 5'-3' exonuclease (e.g., T7 exonuclease or Lambda exonuclease) prior to PCR will only leave intact one strand of DNA ligated to the adapter. In embodiments where all target sequences are of equal lengths, size selection can be used to remove fragments of incorrect lengths during the process of biochemical conversion.

### Example 4.

### The principle of cyclic conversion where three nucleotides are converted per cycle

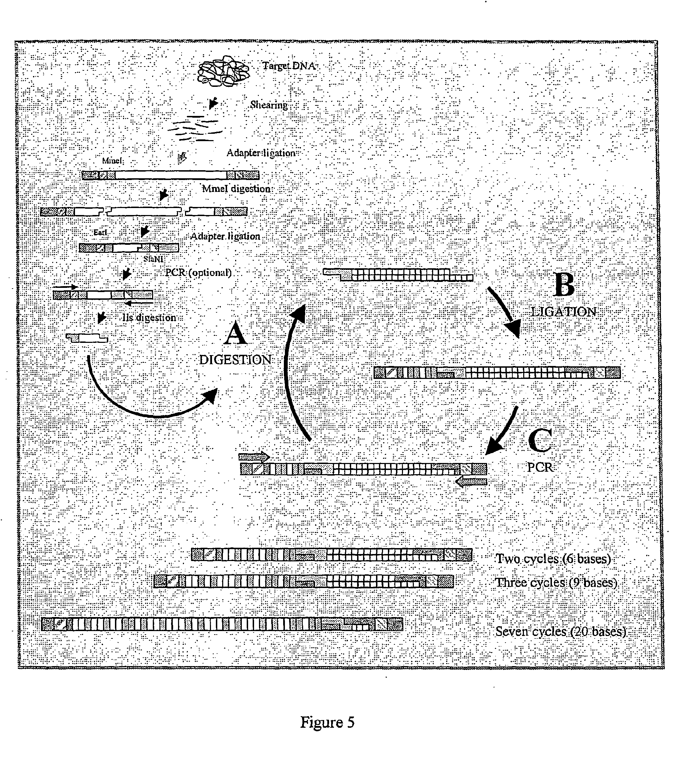

The principle of cyclic conversion of nucleic acid bases into readable signal sequence was demonstrated by converting a sequence of 12 bases in a target DNA into its corresponding signal sequence. The conversion took place in steps through a cyclic method, where 3 bases were converted in each cycle for a total number of 4 cycles. The end product was a signal sequence consisting of 12 signal components representing the relevant base sequence in the target DNA. The experiment is described schematically in Figure 5.

A synthetic target DNA fragment of 66 bp, was generated by annealing the following oligonucleotides: # 003 (5'-PHO-GATCTTGGCTATTCGTCTCTTGGCTTTTCGTCTGATTGTAGACGCCAACG GGACATGATGATGAT-3'; SEQ ID No 12) and # 004 (5'-[PHO-CATCATCATCATCATGTCCCGTTGGCGTCTACAATCAGACGAAAAGCCAA GAGACGAATAGCCAAG-3'; SEQ ID No 13). As evident from the sequence the target molecule contains at the 3' end four consecutive ATG triplets. The annealed molecule corresponds to the nature of those fragments that are obtained after an initial preparation (described in Example 2) in that it has an overhang of 3 bases for signal sequence ligation and an overhang of 4 bases for ligation to a specific adapter.

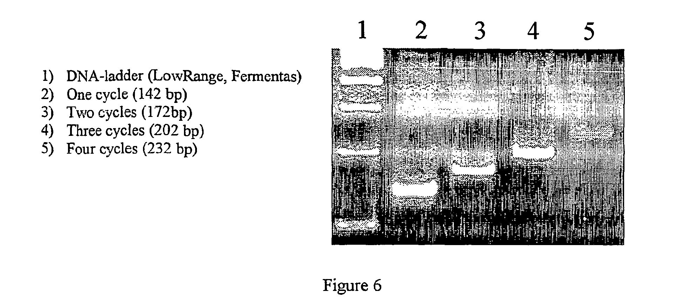

The conversion step where the selection marker is attached (Figure 5, B) was carried out by ligating 1 pmol of the fragment with the specific adapter and its associated signal sequence: 50 mM Tris-HCl, 10 mM MgCl₂, 10 mM DTT, 1 mM ATP, 25 µg/ml BSA, pH 7.5 (ligase buffer, New England Biolabs), 1 pmol adapter # 005 (5'-GATGTAGATGCACTCCCGGACCTC-3'; SEQ ID No 14 annealed to # 006 5'-GAGGTCCGGGAGTGCATCTA-3'; SEQ ID No 15) (MWG-biotech), 1 pmol signal seqeunce # 001 (5'-TGTGTCCGCGTGGCTCTTCTGATCTTGGCTTTTCGTCTCTTGGCTATTCG TCT-3'; SEQ ID No 16, annealed to # 002 5'-PHO-ATCAGACGAATAGCCAAGAGACGAAAAGCCAAGATCAGAAGAGCCACGC GGACACA-3'; SEQ ID No 17) (MWG-biotech), 100 units T4 DNA ligase (New England Biolabs). The incubation took place at 22°C in a PTC-200 thermal cycler (MJResearch) for 1 hour. Selection and amplification of those molecules being successfully ligated to both the adapter and the signal sequence adapter were conducted using PCR under the following conditions (50µl): 10 mM KCI, 10 mM (NH₄)SO₄, 20 mM Tris-HCl, 0.1 % Triton-X-100, pH 8.8 (Thermopol buffer, New England Biolabs), 2 mM Mg²⁺, 200 µM dNTPs (Amersham Bioscience), 10 pmol adapter primer # 006 (5'-GAGGTCCGGGAGTGCATCTA-3'; SEQ ID No 18) (MWG-Biotech) and 10 pmol signal chain primer # 007 (5'-TGTGTCCGCGTGGCTCTTCT-3'; SEQ ID No 19) (MWG-Biotech), approx. 1 pmol ligated target DNA and 0.2 U Vent polymerase (New England Biolabs). PCR cycling was conducted with a PTC-200 (MJReasearch). Hot start: 95°C, 2 minutes, 25 cycles consisting of: 95°C, 15 seconds, 59.3°C, 20 seconds, 72°C, 20 seconds. Complete extension step: 72°C, 60 seconds. Well 2 in Figure 6 shows the results of the initial cycle of the sequencing method. The correct fragment of 142 bp was generated.

### Cycle 2:

In order to convert the next 3 bases, the PCR product was cleaned using a MinElute PCR clean-up kit (Qiagen) and then cut with Eam1104I and SfaNl. To increase the efficiency of the cutting reaction, the cuts were conducted serially under optimal cutting conditions. Eam1104I cutting: 33 mM Tris-Acetate, 10 mM magnesium-acetate, 66 mM potassium-acetate, 0.1 mg/ml BSA, pH 7.9 (Tango Y⁺, Fermentas), 0.5 µg PCR fragment, 20 units Eam1104I in a 25µl reaction. Incubated at 37°C for 1 hour. A Micro Bio-Spin 6 column (BioRad) was used to clean the fragment. SfaNl cutting: 100 mM NaCl, 50 mM Tris-HCl, 10 mM MgCl₂, 1 mM DTT, pH 7.9 (NEB3, New England Biolabs), 0.5 µg PCR fragment, 2.5 units SfaNl. Incubation at 37°C, for 15 minutes in a 50µl reaction.

The digested product was cleaned by PAGE (PolyAcrylamide Gel Electrophoresis) and purified from the gel using a crush and soak method. Gel particles were removed using a Micro Bio-Spin 6 column and the DNA purified using a MinElute Reaction clean-up column (Qiagen).

The conversion step was conducted corresponding to the initial cycle using the same signal sequence and its associated specific adapter. The rest of the procedure followed the same pattern as described for cycle 1 except that an agarose gel and a gel purification kit (Qiaexll, Qiagen) was used to purify the sample after digestion. The result of the PCR amplification in the second cycle is shown in Well 3 in Figure 6. The correct fragment of 172 bp was generated.

Cycle 3 and 4 followed the same pattern as described for cycle 2. The result of the PCR amplification is shown in Wells 4 and 5 where the correct fragments of 202 bp and 232 bp were generated.

## Claims

1. A method for determining the sequence of a target polynucleotide, comprising the steps of:
(i) treating a sample of a double-stranded target polynucleotide to create overhangs at each end, one of which is to be sequenced, each overhang having a defined number of bases;
(ii) dividing the sample into reaction compartments and contacting each separate sample with a different double-stranded polynucleotide signal sequence and a corresponding double-stranded adapter polynucleotide, each signal sequence representing a specific polynucleotide sequence of the same length as that of the overhang to be sequenced, and comprising an overhang that permits hybridisation and ligation to the end of the target polynucleotide opposite that being sequenced, and each adapter comprising an overhang that is of complementary sequence to the sequence represented by the signal sequence, wherein the adapter will hybridise to the overhang on the target sample that is to be sequenced only if the sequence of the adapter overhang is complementary to the overhang that is being sequenced;
(iii) carrying out the polymerase reaction on the sample(s) using primers that hybridise at the ends of the signal sequence and adapter sequence, wherein the product of the polymerase reaction comprises a restriction site that permits cleavage of the adapter to form a new overhang to be sequenced optionally by repeating steps (i) to (iii) using restriction enzymes to create the overhangs; and
(iv) identifying which signal sequences are present on the amplified products, and in which order, to thereby determine the sequence of the target polynucleotide.

2. A method according to claim 1, wherein the overhang that ligates to the signal sequence is at least 3 bases.

3. A method according to claim 1 or claim 2, wherein the overhang to be sequenced is 4 bases.

4. A method according to any preceding claim, wherein the combination of all the sequences represented by the signal sequence in each reaction compartment corresponds to all of the permutations of a sequence comprising the number of bases in the overhang to be sequenced.

5. A method according to any preceding claim, wherein the restriction site is specific for a class IIs enzyme.

6. A method according to claim 5, wherein the enzyme is SfaNl or Earl.

7. A method according to any preceding claim, wherein the polymerase reaction is carried out using methyl-dCTP as a replacement for dCTP.

8. A method according to any preceding claim, wherein the adapter is immobilised on a support material.

9. A method according to any preceding claim, wherein sequential signal sequences and optionally sequential adapters comprise recognition sites for different oligonucleotide primers.

## Patentansprüche

1. Methode für das Bestimmen der Sequenz eines Target-Polynukleotids, umfassend die Schritte des:
(i) Behandelns einer Probe eines doppelstrangingen Target-Polynukleotids, um Überhange an jedem Ende zu bilden, von denen einer sequenziert werden soll, wobei jeder Überhang eine festgelegte Anzahl von Basen aufweist;
(ii) Teilens der Probe in Reaktionskompartimente und Kontaktierens jeder einzelnen Probe mit einer anderen doppelstrangigen Polynukleotidsignalsequenz und einem entsprechenden doppelstrangigen Adapter-Polynukleotid, wobei jede Signalsequenz eine spezifische Polynukleotidsequenz derselben Länge wie diejenigen des zu sequenzierenden Überhangs darstellt und einen Überhang umfasst, der die Hybridisierung und Ligierung an das Ende des Target-Polynukleotids demjenigen gegenüber, das sequenziert wird, erlaubt und wobei jeder Adapter eine Überhang umfasst, der aus einer komplementären Sequenz zu der Sequenz besteht, die durch die Signalsequenz dargestellt ist, wobei der Adapter zu dem Überhang an der Targetprobe hybridisieren wird, der nur dann sequenziert werden soll, wenn die Sequenz des Adapterüberhangs dem Überhang, der sequenziert wird, komplementär ist;
(iii) Durchführens der Polymerasereaktion an der/den Probe(n) unter Anwendung von Primern, die an den Enden der Signalsequenz und Adaptersequenz hybridisieren, wobei das Produkt der Polymerasereaktion eine Restriktionsstelle umfasst, die die Spaltung des Adapters unter Bildung eines neuen Überhangs gestattet, der wahlweise durch die Wiederholung der Schritten (i) bis (iii) unter Anwendung von Restriktionsenzymen unter Bildung der Überhänge sequenziert werden soll; und
(iv) Identifizierens, welche Signalsequenzen an den amplifizierten Produkten vorliegen und in welcher Reihenfolge, um dadurch die Sequenz des Target-Nukleotids zu bestimmen.

2. Methode nach Anspruch 1, wobei der Überhang, der an die Signalsequenz ligiert, mindestens 3 Basen umfasst.

3. Methode nach Anspruch 1 oder Anspruch 2, wobei der zu sequenzierende Überhang 4 Basen umfasst.

4. Methode nach einem der vorhergehenden Ansprüche, wobei die Kombination aller der Sequenzen, die durch die Signalsequenz in jedem Reaktionskompartiment dargestellt sind, allen Permutationen einer Sequenz entspricht, die die Anzahl von Basen in dem zu sequenzierenden Überhang umfasst.

5. Methode nach einem der vorhergehenden Ansprüche, wobei der Restriktionsort für ein Enzym der Klasse IIS spezifisch ist.

6. Methode nach Anspruch 5, wobei das Enzym SfaNI oder Earl ist.

7. Methode nach einem der vorhergehenden Ansprüche, wobei die Polymerasereaktion unter Anwendung von Methyl-dCTP als Ersatz für dCTP durchgeführt wird.

8. Methode nach einem der vorhergehenden Ansprüche, wobei der Adapter auf einem Trägermaterial immobilisiert ist.

9. Methode nach einem der vorhergehenden Ansprüche, wobei sequentielle Signalsequenzen und wahlweise sequentielle Adapter Erkennungsstellen für verschiedene Oligonukleotidprimer umfassen.

## Revendications

1. Méthode de détermination de la séquence d'un polynucléotide cible, comprenant les étapes qui consistent à :
(i) traiter un échantillon d'un polynucléotide cible bicaténaire afin de créer des extensions à chaque extrémité, dont l'une doit être séquencée, chaque extension ayant un nombre défini de bases ;
(ii) diviser l'échantillon en compartiments de réaction et à placer chaque échantillon séparé au contact d'une séquence-signal polynucléotidique bicaténaire différente et un polynucléotide adaptateur bicaténaire correspondant, chaque séquence-signal représentant une séquence polynucléotidique spécifique de longueur égale à celle de l'extension à séquencer, et comprenant une extension qui permet l'hybridation et la ligature à l'extrémité du polynucléotide cible opposé à celui en train d'être séquencé, et chaque adaptateur comprenant une extension dont la séquence complète la séquence représentée par la séquence-signal, dans laquelle l'adaptateur s'hybridera avec l'extension sur l'échantillon cible qui doit être séquencée uniquement si la séquence de l'extension de l'adaptateur est complémentaire de l'extension en train d'être séquencée ;
(iii) effectuer la réaction de la polymérase sur l'échantillon ou les échantillons, en utilisant des amorces qui s'hydrident avec les extrémités de la séquence-signal et de la séquence de l'adaptateur, dans laquelle le produit de la réaction de la polymérase comprend un site de restriction qui permet le clivage de l'adaptateur afin de former une nouvelle extension à séquencer en option en répétant les étapes (i) à (iii) et en utilisant des enzymes de restriction pour créer les extensions ; et
(iv) identifier les séquences-signal qui sont présentes dans les produits amplifiés, et dans quel ordre, pour déterminer ainsi la séquence du polynucléotide cible.

2. Méthode selon la revendication 1, dans laquelle l'extension qui forme une ligature avec la séquence-signal possède au moins 3 bases.

3. Méthode selon la revendication 1 ou 2, dans laquelle l'extension à séquencer possède 4 bases.

4. Méthode selon l'une quelconque des revendications précédentes, dans laquelle l'association de toutes les séquences représentées par la séquence-signal dans chaque compartiment de réaction correspond à toutes les permutations d'une séquence comprenant le nombre de bases dans l'extension à séquencer.

5. Méthode selon l'une quelconque des revendications précédentes, dans laquelle le site de restriction est spécifique à une enzyme de classe IIS.

6. Méthode selon la revendication 5, dans laquelle l'enzyme est SfaNl ou Earl.

7. Méthode selon l'une quelconque des revendications précédentes, dans laquelle la réaction de la polymérase est effectuée en utilisant le méthyl-dCTP à titre de remplacement du dCTP.

8. Méthode selon l'une quelconque des revendications précédentes, dans laquelle l'adaptateur est immobilisé sur un matériau-support.

9. Méthode selon l'une quelconque des revendications précédentes, dans laquelle les séquences-signal séquentielles et les adaptateurs optionnellement séquentiels comprennent des sites de reconnaissance pour diverses amorces oligonucléotidiques.
